# EUROPEAN PATENT APPLICATION

(11) **EP 4 353 736 A1**
(43) Date of publication of application: **17.04.2024**
(21) Application number: 22820609.0
(22) Date of filing: 10.06.2022
(51) Int. Cl.: C07K 14/00, C07K 7/08, C12N 15/62, C07K 16/32, A61P 35/00, A61P 29/00, A61K 38/00

(54) **BIO PROTAC PROTEIN HAVING INTRACELLULAR DELIVERY FUNCTION, AND PHARMACEUTICAL COMPOSITION COMPRISING SAME**

(30) Priority: 11.06.2021 KR 20210075839
(71) Applicant: Nibec Co., Ltd., Chungcheongbuk-do 27816 (KR); Seoul National University R&DB Foundation, Seoul 08826 (KR)
(72) Inventor: PARK, Yoon Jeong, Seoul 08291 (KR); CHUNG, Chong-Pyoung, Seoul 05618 (KR); LEE, Jue-Yeon, Gwacheon-si, Gyeonggi-do 13831 (KR); YOON, Gook-Jin, Seoul 05659 (KR)
(74) Representative: Mewburn Ellis LLP
(86) International application number: PCT/KR2022/008231
(87) International publication number: WO 2022/260482

(57) **Abstract**

The present invention relates to a proteolysis targeting chimera (PROTAC) protein having an intracellular delivery function, and a pharmaceutical composition comprising same. The PROTAC protein according to the present invention has higher solubility than a PROTAC prepared by a conventional method and efficiently degrades intrinsic disease proteins when applied to cells, and thus is effective in the treatment of cancer or inflammatory diseases.

## Description

### Technical Field

The present invention relates to a proteolysis targeting chimera (PROTAC) protein having an intracellular delivery function and a pharmaceutical composition comprising the same.

### Background Art

Proteolysis targeting chimera (PROTAC) is a doubleheaded molecule that binds to a disease-causing protein, inducing the proteasome to degrade the protein, resulting in selective proteolysis. PROTAC is composed of two protein-binding molecules, one for binding to an E3 ubiquitin ligase and the remaining one for binding to a target protein. By binding to both proteins, PROTAC delivers the target protein to the E3 ligase and causes tagging, namely ubiquitination, of the target protein for subsequent degradation by the proteasome.

Ubiquitination involves three steps: activation, conjugation, and ligation performed by ubiquitin-activating enzymes (E1), ubiquitin-conjugating enzymes (E2), and ubiquitin ligases (E3). As a result of this sequential cascade, ubiquitin is covalently attached to the target protein. The ubiquitinated protein is eventually degraded by the proteasome.

PROTAC technology was first reported in 2001 (Sakamoto et al., Proceedings of the National Academy of Sciences of the United States of America. 98: 8554, 2001). Since then, this technology has been used in various drug designs: pVHL, MDM2, beta-TrCP1, cereblon, and c-IAP1. PROTAC drugs have been developed mostly from small-molecule compounds. Various types of small-molecule compounds derived from the thalidomide family have the advantage of being able to simultaneously conjugate a target protein and an E3 ligase structurally. These known PROTAC drugs are very useful, but measures are needed to address concerns about the low solubility of small-molecule compounds, the decreased intrinsic protein degradation efficiency due to low intracellular penetration, and safety, and a need for PROTAC drugs that overcome these problems is required.

Therefore, the present inventors have made great efforts to develop a PROTAC protein with high target protein degradation efficiency and intracellular delivery function, rather than using a small-molecule compound, and ascertained that a target protein binding sequence linked via a linker composed of five glycines to a von Hippel-Lindau (VHL) protein sequence as a substrate recognition unit capable of transferring, to a target protein, an E3 ligase, which is an important target in cancer, inflammatory diseases, and infection, may be constructed, and an antibody or a cell-penetrating peptide may be attached to the end of this chimeric protein using a linker, so the target protein of the underlying disease may be actually degraded through intracellular delivery, thus culminating in the present invention.

The above information described in the background section is only for improving the understanding of the background of the present invention, and it does not include information forming the prior art known to those of ordinary skill in the art to which the present invention pertains.

### Summary of the Invention

It is one object of the present invention to provide a PROTAC protein having high target protein degradation efficiency and intracellular delivery function.

It is another object of the present invention to provide a nucleic acid encoding the PROTAC protein.

It is still another object of the present invention to provide a pharmaceutical composition comprising the PROTAC protein or the nucleic acid encoding the same as an active ingredient.

It is yet another object of the present invention to provide a method of preventing or treating a disease comprising administering the PROTAC protein or the nucleic acid encoding the same.

It is still yet another object of the present invention to provide the use of the PROTAC protein or the nucleic acid encoding the same for the prevention or treatment of a disease.

It is even yet another object of the present invention to provide the use of the PROTAC protein or the nucleic acid encoding the same in the manufacture of a medicament for preventing or treating a disease.

In order to accomplish the above objects, the present invention provides a PROTAC protein having the structure of Chemical Formula 1 or Chemical Formula 2 below:

[Chemical Formula 1] PEP-[L₁-(TB-L₂-UR)ₙ]ₘ

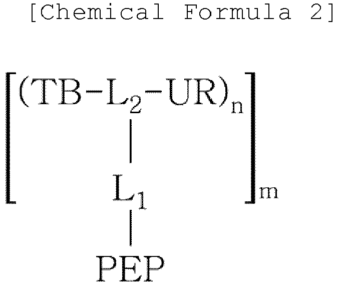

in Chemical Formula 1 or Chemical Formula 2,
(i) PEP is an antibody or an antibody fragment, or a cell-penetrating peptide,
(ii) L₁ and L₂ are linkers, L₁ and L₂ may be the same as or different from each other, and L₁ binds to TB or L₂,
(iii) TB is a binder or conjugate that binds to a target protein,
(iv) UR is a ligand binding to a ubiquitin ligase, and
(v) n and m are each independently an integer of 1 to 10.

In addition, the present invention provides a nucleic acid encoding the PROTAC protein.

In addition, the present invention provides a pharmaceutical composition comprising the PROTAC protein or the nucleic acid encoding the same as an active ingredient.

In addition, the present invention provides a method of preventing or treating a disease comprising administering the PROTAC protein or the nucleic acid encoding the same.

In addition, the present invention provides the use of the PROTAC protein or the nucleic acid encoding the same for the prevention or treatment of a disease.

In addition, the present invention provides the use of the PROTAC protein or the nucleic acid encoding the same in the manufacture of a medicament for preventing or treating a disease.

### Brief Description of Drawings

FIG. 1 schematically shows an expression vector of a PROTAC protein with cell-penetrating activity.
FIG. 2 shows results of Western blotting of the PROTAC protein expressed on a plasmid introduced into cells.
FIG. 3 shows results of cancer cell viability by the PROTAC protein expressed on the plasmid introduced into cells.
FIG. 4 shows results of Western blotting confirming inhibition of expression of active KRAS by the PROTAC protein expressed on the plasmid introduced into cells.
FIG. 5 shows results of Western blotting confirming expression of the PROTAC protein and inhibition of expression of active KRAS in cells after introduction of mRNA encoding the PROTAC protein into the cells.

### Detailed Description and Preferred Embodiments of the Invention

Unless otherwise defined, all technical and scientific terms used herein have the same meanings as typically understood by those skilled in the art to which the present invention belongs. In general, the nomenclature used herein and experimental methods described below are well known in the art and are typical.

The present invention is based on the discovery that a ubiquitin pathway protein ubiquitinates any target protein when the ubiquitin pathway protein and the target protein are in proximity to a chimeric construct that binds to the ubiquitin pathway protein and the target protein.

The present invention is mainly intended to overcome limitations of conventional PROTAC technology in that PROTAC has low solubility or does not efficiently degrade intrinsic disease proteins when applied to cells.

In one aspect, the present invention is directed to a PROTAC protein having the structure of Chemical Formula 1 or Chemical Formula 2 below:

[Chemical Formula 1] PEP-[L₁-(TB-L₂-UR)ₙ]ₘ

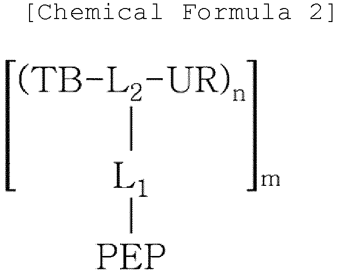

in Chemical Formula 1 or Chemical Formula 2,
(i) PEP is an antibody or an antibody fragment, or a cell-penetrating peptide,
(ii) L₁ and L₂ are linkers, L₁ and L₂ may be the same as or different from each other, and L₁ binds to TB or L₂,
(iii) TB is a binder or conjugate that binds to a target protein,
(iv) UR is a ligand binding to a ubiquitin ligase, and
(v) n and m are each independently an integer of 1 to 10.

As used herein, the term "PROTAC" is an abbreviation of proteolysis targeting chimera, refers to a bifunctional small molecule composed of two active domains and a linker, and is capable of removing a specific unwanted protein. In the present specification, "PROTAC protein" may be used interchangeably with "bio PROTAC protein" and "chimeric protein".

The PROTAC protein according to the present invention is capable of inducing ubiquitination of a selected target protein, and is designed to be linked via a linker to include a target protein binding site and a ubiquitin ligase binding site.

In the present invention, the PROTAC protein includes a ubiquitin ligase binding ligand at one end and a target protein binding site at the remaining end, and a cell-penetrating domain is included in the end of the protein binding to the target protein or in the linker that connects the ubiquitin ligase binding ligand and the target protein binding site to each other. Thereby, when the PROTAC protein is introduced into cells, it is located close to the target protein, making it possible to induce degradation of the protein, and is present in high concentration in the cells depending on the numerical values of n and m, inducing effective target protein degradation.

In the present invention, the PROTAC protein may be represented by the amino acid sequences of SEQ ID NOs: 1 to 6, but is not limited thereto.
SEQ ID NO: 1: 
EVQLLESGGGLVQPGGSLRLSCAASGFTFSTFSMNWVRQAPGKGLEWVSYI
SRTSKTIYYADSVKGRFTISRDNSKNTLYLQMNSLRAEDTAVYYCARGRFFDYWGQGT
LVTVSSGGSEGKSSGSGSESKSTGGSDIQMTQSPSSLSASVGDRVTITCRASQSISSY
LNWYQQKPGEAPKLLIYSASVLQSGVPSRFSGSGSGTDFTLTISSLQPEDFATYYCQQ
SVMIPMTFGQGTKVEIKGSGGGGSMPRRAENWDEAEVGAEEAGVEEYGPEEDGGEESG
AEESGPEESGPEELGAEEEMEAGRPRPVLRSVNSREPSQVIFCNRSPRVVLPVWLNFD
GEPQPYPTLPPGTGRRIHSYRGHLWLFRDAGTHDGLLVNQTELFVPSLNVDGQPIFAN
ITLPVYTLKERCLQVVRSLVKPENYRRLDIVRSLYEDLEDHPNVQKDLERLTQERIAH
QRMGDENLYFQGGSGGSGGSHHHHHHHH
SEQ ID NO: 2: 
MAWVWTLLFLMAAAQSIQAVSRRRRRGGRRRRGGGGSEVQLLESGGGLVQ

PGGSLRLSCAASGFTFSTFSMNWVRQAPGKGLEWVSYISRTSKTIYYADSVKGRFTIS

RDNSKNTLYLQMNSLRAEDTAVYYCARGRFFDYWGQGTLVTVSSGGSEGKSSGSGSES

KSTGGSDIQMTQSPSSLSASVGDRVTITCRASQSISSYLNWYQQKPGEAPKLLIYSAS

VLQSGVPSRFSGSGSGTDFTLTISSLQPEDFATYYCQQSVMIPMTFGQGTKVEIKGSG

GGGSMPRRAENWDEAEVGAEEAGVEEYGPEEDGGEESGAEESGPEESGPEELGAEEEM

EAGRPRPVLRSVNSREPSQVIFCNRSPRVVLPVWLNFDGEPQPYPTLPPGTGRRIHSY

RGHLWLFRDAGTHDGLLVNQTELFVPSLNVDGQPIFANITLPVYTLKERCLQVVRSLV

KPENYRRLDIVRSLYEDLEDHPNVQKDLERLTQERIAHQRMGDENLYFQGGSGGSGGS

HHHHHHHH
SEQ ID NO: 3: 
MAWVWTLLFLMAAAQSIQAEVQLLESGGGLVQPGGSLRLSCAASGFTFSTF

SMNWVRQAPGKGLEWVSYISRTSKTIYYADSVKGRFTISRDNSKNTLYLQMNSLRAED
 
TAVYYCARGRFFDYWGQGTLVTVSSGGSEGKSSGSGSESKSTGGSDIQMTQSPSSLSA

SVGDRVTITCRASQSISSYLNWYQQKPGEAPKLLIYSASVLQSGVPSRFSGSGSGTDF

TLTISSLQPEDFATYYCQQSVMIPMTFGQGTKVEIKGSGGGGSMPRRAENWDEAEVGA

EEAGVEEYGPEEDGGEESGAEESGPEESGPEELGAEEEMEAGRPRPVLRSVNSREPSQ

VIFCNRSPRVVLPVWLNFDGEPQPYPTLPPGTGRRIHSYRGHLWLFRDAGTHDGLLVN

QTELFVPSLNVDGQPIFANITLPVYTLKERCLQVVRSLVKPENYRRLDIVRSLYEDLE

DHPNVQKDLERLTQERIAHQRMGDGGGGSVSRRRRRRGGRRRRENLYFQGGSGGSGGS

HHHHHHHH
SEQ ID NO: 4: 
EVQLLESGGGLVQPGGSLRLSCAASGFTFSTFSMNWVRQAPGKGLEWVSYI
SRTSKTIYYADSVKGRFTISRDNSKNTLYLQMNSLRAEDTAVYYCARGRFFDYWGQGT
LVTVSSGGSEGKSSGSGSESKSTGGSDIQMTQSPSSLSASVGDRVTITCRASQSISSY
LNWYQQKPGEAPKLLIYSASVLQSGVPSRFSGSGSGTDFTLTISSLQPEDFATYYCQQ
SVMIPMTFGQGTKVEIKGSGGGGSMENRWQVMIVWQVDRMRIRTWKSLVKHHMYVSGK
ARGWFYRHHYESPHPRISSEVHIPLGDARLVITTYWGLHTGERDWHLGQGVSIEWRKK
RYSTQVDPELADQLIHLYYFDCFSDSAIRKALLGHIVSPRCEYQAGHNKVGSLQYLAL
AALITPKKIKPPLPSVTKLTEDRWNKPQKTKGHRGSHTMNGHENLYFQGGSGGSGGSH
HHHHHH
SEQ ID NO: 5: 
MAWVWTLLFLMAAAQSIQAVSRRRRRRGGRRRRGGGGSEVQLLESGGGLVQ

PGGSLRLSCAASGFTFSTFSMNWVRQAPGKGLEWVSYISRTSKTIYYADSVKGRFTIS

RDNSKNTLYLQMNSLRAEDTAVYYCARGRFFDYWGQGTLVTVSSGGSEGKSSGSGSES

KSTGGSDIQMTQSPSSLSASVGDRVTITCRASQSISSYLNWYQQKPGEAPKLLIYSAS

VLQSGVPSRFSGSGSGTDFTLTISSLQPEDFATYYCQQSVMIPMTFGQGTKVEIKGSG
 
GGGSMENRWQVMIVWQVDRMRIRTWKSLVKHHMYVSGKARGWFYRHHYESPHPRISSE
VHIPLGDARLVITTYWGLHTGERDWHLGQGVSIEWRKKRYSTQVDPELADQLIHLYYF
DCFSDSAIRKALLGHIVSPRCEYQAGHNKVGSLQYLALAALITPKKIKPPLPSVTKLT
EDRWNKPQKTKGHRGSHTMNGHENLYFQGGSGGSGGSHHHHHHHH
SEQ ID NO: 6:

MAWVWTLLFLMAAAQSIQAEVQLLESGGGLVQPGGSLRLSCAASGFTFSTF
SMNWVRQAPGKGLEWVSYISRTSKTIYYADSVKGRFTISRDNSKNTLYLQMNSLRAED
TAVYYCARGRFFDYWGQGTLVTVSSGGSEGKSSGSGSESKSTGGSDIQMTQSPSSLSA
SVGDRVTITCRASQSISSYLNWYQQKPGEAPKLLIYSASVLQSGVPSRFSGSGSGTDF
TLTISSLQPEDFATYYCQQSVMIPMTFGQGTKVEIKGSGGGGSMENRWQVMIVWQVDR
MRIRTWKSLVKHHMYVSGKARGWFYRHHYESPHPRISSEVHIPLGDARLVITTYWGLH
TGERDWHLGQGVSIEWRKKRYSTQVDPELADQLIHLYYFDCFSDSAIRKALLGHIVSP
RCEYQAGHNKVGSLQYLALAALITPKKIKPPLPSVTKLTEDRWNKPQKTKGHRGSHTM
NGHGGGGSVSRRRRRRGGRRRRENLYFQGGSGGSGGSHHHHHHH

The binder or conjugate that binds to the target protein, named TB in the present invention, may be selected from a library. TB is designed to be coupled via a linker with a ubiquitin pathway protein binding site, such as a VHL protein. The ubiquitin pathway protein recognizes the E3 ubiquitin ligase.

In the present invention, the target protein of the PROTAC protein may include, but is not limited to, a mutated RAS superfamily, a kinase, a transcription factor, and a phosphatase.

In the present invention, the RAS superfamily may be selected from the group consisting of KRAS, HRAS, and NRAS.

In the present invention, TB may be selected from the group consisting of a mutated RAS superfamily inhibitor, a kinase inhibitor, a phosphatase inhibitor, a heat shock protein 90 (HSP90) inhibitor, an MDM2 (mouse double minute 2 homolog) inhibitor, an HDAC (histone deacetylase) inhibitor, a human lysine methyltransferase inhibitor, an angiogenesis inhibitor, an immunosuppressive compound, a compound targeting human BET (bromodomain and extraterminal domain) bromodomain-containing protein, a compound targeting aryl hydrocarbon receptor (AHR), a compound targeting EGF (epithelial growth factor) receptor kinase, a compound targeting FKBP (FK506 binding protein), a compound targeting androgen receptor (AR), a compound targeting estrogen receptor (ER), a compound targeting thyroid hormone receptor, a compound targeting HIV protease, a compound targeting HIV integrase, a compound targeting HCV protease, a compound targeting acyl-protein thioesterase-1 (APT1), and a compound targeting acyl-protein thioesterase-2 (APT2), but is not limited thereto.

In the present invention, TB may be a peptide comprising any one amino acid selected from SEQ ID NO: 7 to SEQ ID NO: 14, but is not limited thereto.
SEQ ID NO: 7: LTPHKHHKHLHA
SEQ ID NO: 8: WPGKHHNHYLRS
SEQ ID NO: 9: HDGYWWHSMTMW
SEQ ID NO: 10: LIHPMTVKHVHL
SEQ ID NO: 11: GSHWHFPKHQQQ
SEQ ID NO: 12: GSHWHFPKHQQH
SEQ ID NO: 13: WPGKHHHHYLRR
SEQ ID NO: 14:

EVQLLESGGGLVQPGGSLRLSCAASGFTFSTFSMNWVRQAPGKGLEWVSYI
SRTSKTIYYADSVKGRFTISRDNSKNTLYLQMNSLRAEDTAVYYCARGRFFDYWGQGT
LVTVSSGGSEGKSSGSGSESKSTGGSDIQMTQSPSSLSASVGDRVTITCRASQSISSY
LNWYQQKPGEAPKLLIYSASVLQSGVPSRFSGSGSGTDFTLTISSLQPEDFATYYCQQ
SVMIPMTFGQGTKVEIK

In the present invention, UR is a ligand binding to a ubiquitin ligase, and may be a ligand binding to an E3 ligase selected from the group consisting of XIAP (X-linked inhibitor of apoptosis protein), VHL (von Hippel-Lindau) protein, IAPs (inhibitor of apoptosis proteins), cereblon, and MDM2 (mouse double minute 2 homolog), but is not limited thereto.

In the present invention, PEP may be an antibody or an antibody fragment, or a cell-penetrating peptide.

In the present invention, the cell-penetrating peptide is a peptide having specific ability to penetrate target disease cells, and examples of the usable cell-penetrating peptide sequence are shown in Table 1 below, but are not limited thereto.

**[Table 1]**

| Sequence of cancer cell-penetrating peptide | | | | |
|---|---|---|---|---|
| Type and location of spacer | Cancer cell-penetrating peptide (CCPP) | Amino acid sequence of spacer and cancer cell-penetrating peptide | SEQ ID NO: | Protein connection site |
| N term of (CCPPGGGGS)o* | H4K | GGGGS-HRRCNKNNKKR | 15 | C term |
| | H4P | GGGGS-HRRCNPNNKKR | 16 | C term |
| | LMWP | GGGGS-VSRRRRRRGGRRRR | 17 | C term |
| | hBD3-3 | GGGGS-GKCSTRGRKCCRRKK | 18 | C term |
| | NRP | GGGGS-NRPDSAQFWLHHRR | 19 | |
| C term of (CCPPGGGGS)o* | H4K | HRRCNKNNKKR-GGGGS | 20 | N term |
| | H4P | HRRCNPNNKKR-GGGGS | 21 | N term |
| | LMWP | VSRRRRRRGGRRRR-GGGGS | 22 | N term |
| | hBD3-3 | GKCSTRGRKCCRRKK-GGGGS | 23 | N term |
| | NRP | NRPDSAQFWLHHRRR-GGGGS | 24 | |

| | | | | |
|---|---|---|---|---|
| (*: o is defined as 1-5 and determines the length of the spacer) | | | | |

In the present invention, the antibody may bind to at least one polypeptide selected from the group consisting of EGFR, DLL3, EDAR, CLL1, BMPR1B, E16, STEAP1, 0772P, MPF, NaPi2b, Sema 5b, PSCA hlg, ETBR, MSG783, STEAP2, TrpM4, CRIPTO, CD21, CD79b, FcRH2, B7-H4, HER2, NCA, MDP, IL20Rct, brevican, EphB2R, ASLG659, PSCA, GEDA, BAFF-R, CD22, CD79a, CXCRS, HLA-DOB, P2X5, CD72, LY64, FcRH1, IRTA2, TENB2, PMEL17, TMEFF1, GDNF-Ra1, Ly6E, TMEM46, Ly6G6D, LGR5, RET, LY6K, GPR19, GPR54, ASPHD1, tyrosinase, TMEM118, GPR172A, MUC16, and CD33, but the present invention is not limited thereto.

In the present invention, the antibody may be a monoclonal antibody or a variant thereof, and the monoclonal antibody may be selected from the group consisting of trastuzumab, cetuximab, rituximab, brentuximab, gemtuzumab, inotuzumab, sacituzumab, alemtuzumab, and nimotuzumab, but is not limited thereto.

In another aspect, the present invention is directed to a nucleic acid encoding the PROTAC protein.

In the present invention, the nucleic acid may be DNA, mRNA, plasmid DNA, etc., and the gene encoding the PROTAC protein, such as plasmid DNA or mRNA derived therefrom, may be delivered into cells by a cell-penetrating nanocarrier, thus exhibiting the same target protein degradation efficacy.

In the present invention, the nucleic acid may be used along with various carriers such as lipid nanoparticles (LNPs), liposomes, etc., which are known to effectively deliver oligonucleotides into cells, but the present invention is not limited thereto.

In still another aspect, the present invention is directed to a pharmaceutical composition comprising the PROTAC protein or the nucleic acid encoding the PROTAC protein.

The present invention is directed to degradation of the target protein and thus provides a wide range of pharmaceutical compositions related to the PROTAC protein according to the present invention.

In the present invention, the pharmaceutical composition may be used for the treatment or prevention of cancer or an inflammatory disease, particularly a disease selected from the group consisting of cancer, asthma, autoimmune disease, rheumatoid arthritis, multiple sclerosis, ciliary disease, cleft palate, diabetes, heart disease, hypertension, inflammatory bowel disease, mental retardation, mood disorder, obesity, refractive error, infertility, Engelman syndrome, Canavan disease, chronic digestive disorder, Charcot-Marie-Tooth disease, cystic fibrosis, Duchenne muscular dystrophy, hemochromatosis, hemophilia, Klinefelter syndrome, neurofibromatosis, phenylketonuria, autosomal dominant polycystic neoplasm (PKD1 or PKD2), Prader-Willi syndrome, sickle cell anemia, Tay-Sachs disease, Turner syndrome, HIV-infected disease, and HCV-infected disease.

In the present invention, the cancer may be selected from the group consisting of squamous cell carcinoma, basal cell carcinoma, adenocarcinoma, hepatocellular carcinoma, renal cell carcinoma, bladder cancer, bowel cancer, breast cancer, cervical cancer, uterine cancer, colon cancer, esophageal cancer, head cancer, kidney cancer, liver cancer, lung cancer, ovary cancer, pancreatic cancer, prostate cancer, gastric cancer, leukemia, benign and malignant lymphoma, especially Burkitt lymphoma and non-Hodgkin lymphoma, benign and malignant melanoma, myeloproliferative disease, sarcoma including Ewing sarcoma, angiosarcoma, Kaposi sarcoma, liposarcoma, myoma, neuroepithelial sarcoma, synovial sarcoma, neurosarcoma, astrocytoma, oligodendrogliocytoma, ependymoma, glioblastoma, neuroblastoma, gangliocytoma, ganglioglioma, medulloblastoma, pineocytoma, meningioma, meningeal sarcoma, neurofibroma, and schwannoma, testicular cancer, thyroid cancer, carcinosarcoma, Hodgkin disease, Wilms tumor, and teratocalcinomas.

In the present invention, the inflammatory disease may be selected from the group consisting of arthritis, autoimmune disease, Parkinson's disease, dementia, hepatitis, and viral infection.

The pharmaceutical composition of the present invention may be administered via oral, parenteral, inhalation spray, topical, rectal, nasal, or implanted reservoir routes, and may be administered using nanoparticles or liposomes as a carrier upon oral or parenteral administration.

In an embodiment of the present invention, a single dose of the PROTAC protein may be 1 ng/kg to 100 mg/kg, preferably 5 ng/kg to 50 mg/kg, and administration may be performed once a day or 1-3 times a week. However, the dose and interval of administration are not limited thereto.

In yet another aspect, the present invention is directed to a method of preventing or treating a disease comprising administering the PROTAC protein or the nucleic acid encoding the PROTAC protein.

In still yet another aspect, the present invention is directed to the use of the PROTAC protein or the nucleic acid encoding the PROTAC protein for the prevention or treatment of a disease.

In even yet another aspect, the present invention is directed to the use of the PROTAC protein or the nucleic acid encoding the PROTAC protein in the manufacture of a medicament for preventing or treating a disease.

In the present invention, the disease may be cancer or an inflammatory disease, but is not limited thereto.

In the present invention, the method and use comprise the PROTAC protein or the nucleic acid encoding the same according to the present invention, and relate to the pharmaceutical composition comprising the PROTAC protein or the nucleic acid encoding the same, so a redundant description of the PROTAC protein or the nucleic acid encoding the same and the pharmaceutical composition is omitted.

A better understanding of the present invention may be obtained through the following examples. These examples are merely set forth to illustrate the present invention, and are not to be construed as limiting the scope of the present invention, as will be apparent to those of ordinary skill in the art.

### Example 1: Expression of PROTAC protein with cell-penetrating activity in animal cells (mammalian cells)

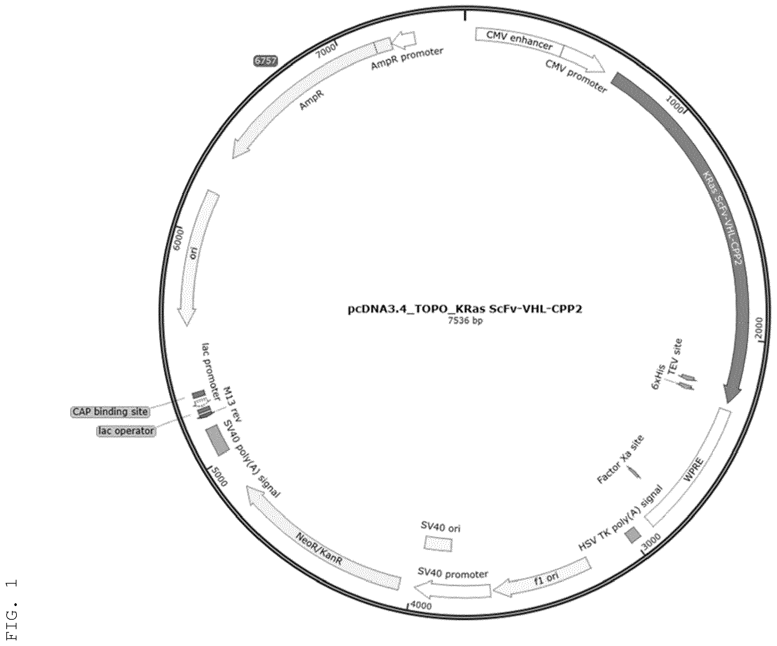

The present inventors designed an expression vector of a PROTAC protein that degrades a target protein as described below. A monoclonal antibody targeting an activated protein (KRAS^{G12C}) in which the 12^{th} glycine of human KRAS is mutated to cysteine, a VHL protein binding to an E3 ligase, and cDNA including a kozak sequence that binds to ribosome and helps transcription were inserted into a pcDNA3.4 vector, cloned, and identified by DNA sequencing. To the resulting plasmid DNA sequence, a signal peptide, a linker, and a PEP sequence were added. The signal peptide was located at the N-terminus, and the monoclonal antibody targeting mutant human KRAS G12C, VHL, and PEP linked by linkers of various lengths were cloned in various locations. A CHO-S cell line was transfected with the plasmid thus constructed (FIG. 1), followed by culture for 7 days with addition of an enhancer.

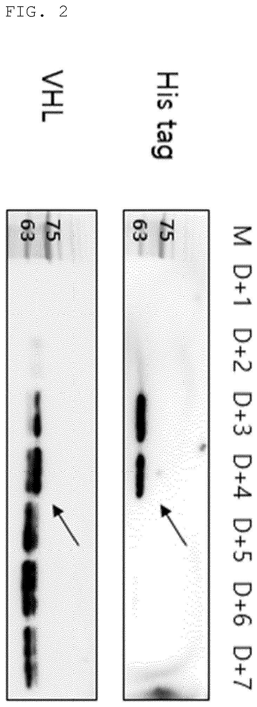

The expressed PROTAC protein was measured by Western blotting using anti-His tag antibody (Abcam, ab18184) and anti-VHL antibody (Invitrogen, MA5-13940), and the results of Western blotting are shown in FIG. 2. The theoretical protein size is 56 kDa, and the PROTAC protein began to appear from the 3^{rd} day of culture. His tag did not appear after 5 days of culture, indicating that it was cleaved from the protein.

### Example 2: Confirmation of cancer cell growth inhibitory effect by PROTAC protein

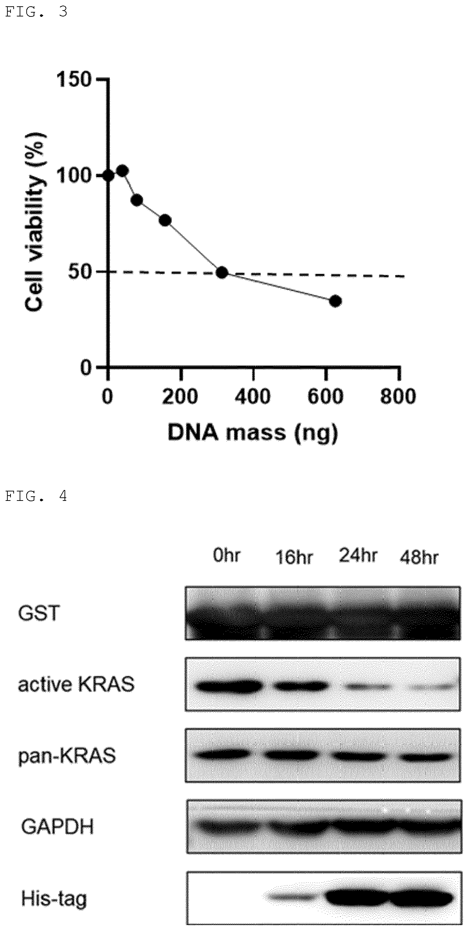

Cancer cells were transfected with a plasmid encoding PROTAC, and an MTT assay was performed to evaluate the anticancer effect of the PROTAC protein expressed by the plasmid. 5×10³ H358 cells (ATCC, CRL-5807), non-small cell lung cancer cells having the phenotype of human KRAS^{G12C}, were transfected with various amounts of plasmid using Lipofectamine 3000 (Invitrogen, L3000001), and after 72 hours, cell viability was evaluated through MTT assay (FIG. 3). H358 cell viability decreased with an increase in the concentration of the treated plasmid. Therefore, it was confirmed that the PROTAC protein was expressed by the plasmid introduced into the cells and thus acted.

### Example 3: Confirmation of KRAS signaling inhibitory effect by PROTAC protein

In order to confirm degradation of the target protein by the PROTAC protein expressed in cells, H358 cells (ATCC, CRL-5807), non-small cell lung cancer cells having the phenotype of human KRAS^{G12C}, were transfected with the plasmid DNA constructed in Example 1 using Lipofectamine 3000 (Invitrogen, L3000001). After transfection, the cells were lysed at 16, 24, and 48 hours to obtain cell lysates.

In order to isolate active KRAS, 80 µg of GST-Raf1-RBD was added using an active GTPase kit (Cell Signaling, 11860S), and 500 µg of protein was dispensed into a spin cup. After reaction at 4°C for 1 hour and then centrifugation at 6000x g for 15 seconds, the column was transferred to a new tube, and 400 µl of 1x lysis buffer was added thereto. After centrifugation at 6000x g for 15 seconds, the column was transferred to a new tube, and 50 µl of 2x SDS buffer (a mixture of 200 µl of 5X SDS sample loading dye and 300 µl of water) was dispensed and then allowed to react for 2 minutes. After centrifugation at 6000x g for 2 minutes, heating was performed at 100°C for 7 minutes. Proteins (protein loading volume: 20 µl/lane) were electrophoretically separated by 11% SDS-PAGE and transferred to a nitrocellulose membrane. In order to measure ERK1/2 corresponding to a subsignal of KRAS, 30 µg of protein was electrophoresed by 11% SDS-PAGE and transferred to a nitrocellulose membrane. Blocking was performed for 1 hour with 5% skim milk dissolved in T-TBS, and the primary antibody was diluted 1:1000 in T-TBS and allowed to react overnight while inverting at 4°C. After washing with T-TBS three times for 10 minutes, the secondary antibody was diluted 1:3000 in T-TBS and allowed to react for 1 hour while inverting at room temperature. After washing with T-TBS three times for 10 minutes, chemiluminescence was performed with an ECL substrate (Thermo, 34580) and confirmed with Amersham Imager 680 (GE) . Information on the antibodies used is shown in Table 2 below.

**[Table 2]**

| Antibody information used | | | |
|---|---|---|---|
| | Antibody name | Company | Catalog number |
| 1 | GST | Abeam | ab19256 |
| 2 | Active KRAS | Santa cruz | SC-30 |
| 3 | pan RAS (E4K9L) | Cell signaling | 3965S |
| 4 | anti-His tag antibody | abcam | ab18184 |
| 5 | GAPDH | Santa cruz | sc-47724 |
| 6 | Goat anti-Rabbit IgG | BETHYL | A120-101P |
| 7 | Goat anti-Mouse IgG | BETHYL | A90-116P |

Using immunoblotting, the PROTAC protein was identified with anti-His tag antibody, and pan KRAS or active KRAS in the cells were identified (FIG. 4). As the PROTAC protein was expressed, the expression level of active KRAS decreased, but pan KRAS did not change. Therefore, it was confirmed that the PROTAC protein selectively degraded only active KRAS and did not degrade normal pan KRAS.

### Example 4: Introduction of mRNA encoding PROTAC protein into cells

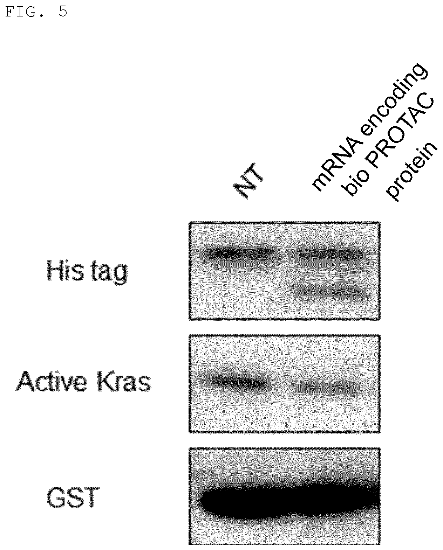

In order to express a PROTAC protein in cells, mRNA was constructed instead of the plasmid. Stability of mRNA was increased by adding 5' capping and 3' poly A tail. Transfection was performed using Lipofectamine MessengerMAX (Invitrogen, LMRNA003). 24 hours after transfection, the cells were lysed to obtain cell lysate. Active KRAS was screened in the same manner as in the method described in Example 3. Using immunoblotting, the PROTAC protein expressed by mRNA introduced into the cells was identified with anti-His tag antibody (Abeam). It was confirmed that the expression level of active KRAS decreased as the PROTAC protein was expressed (FIG. 5). The PROTAC protein introduced in the form of mRNA was expressed and thus active KRAS, which is the target protein, was degraded.

### Industrial Applicability

According to the present invention, a PROTAC protein has high solubility compared to PROTAC constructed by conventional methods, and can efficiently degrade intrinsic disease protein when applied to cells, and is thus effective for the treatment of cancer or an inflammatory disease.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### Sequence List Free Text

An electronic file is attached.

## Claims

1. A PROTAC protein having a structure of Chemical Formula 1 or Chemical Formula 2 below:
[Chemical Formula 1] PEP-[L₁-(TB-L₂-UR)ₙ]ₘ 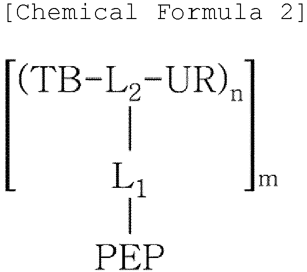
in Chemical Formula 1 or Chemical Formula 2,
(i) PEP is an antibody or an antibody fragment, or a cell-penetrating peptide,
(ii) L₁ and L₂ are linkers, L₁ and L₂ are same as or different from each other, and L₁ binds to TB or L₂,
(iii) TB is a binder or conjugate that binds to a target protein,
(iv) UR is a ligand binding to a ubiquitin ligase, and
(v) n and m are each independently an integer of 1 to 10.

2. The PROTAC protein according to claim 1, wherein the target protein is selected from the group consisting of a mutated RAS superfamily, a kinase, a transcription factor, and a phosphatase.

3. The PROTAC protein according to claim 2, wherein the RAS superfamily is selected from the group consisting of KRAS, HRAS, and NRAS.

4. The PROTAC protein according to claim 1, wherein the TB is selected from the group consisting of a mutated RAS superfamily inhibitor, a kinase inhibitor, a phosphatase inhibitor, a heat shock protein 90 inhibitor, an MDM2 inhibitor, an HDAC inhibitor, a human lysine methyltransferase inhibitor, an angiogenesis inhibitor, an immunosuppressive compound, a compound targeting human BET bromodomain-containing protein, a compound targeting aryl hydrocarbon receptor, a compound targeting EGF (epithelial growth factor) receptor kinase, a compound targeting FKBP, a compound targeting androgen receptor, a compound targeting estrogen receptor, a compound targeting thyroid hormone receptor, a compound targeting HIV protease, a compound targeting HIV integrase, a compound targeting HCV protease, a compound targeting acyl-protein thioesterase-1, and a compound targeting acyl-protein thioesterase-2.

5. The PROTAC protein according to claim 1, wherein the TB is a peptide comprising any one amino acid selected from SEQ ID NO: 7 to SEQ ID NO: 14.

6. The PROTAC protein according to claim 1, wherein the UR is a ligand binding to an E3 ligase selected from the group consisting of XIAP, VHL protein, IAPs, cereblon, and MDM2.

7. The PROTAC protein according to claim 1, wherein the antibody is an antibody or a fragment thereof binding to at least one polypeptide selected from the group consisting of EGFR, DLL3, EDAR, CLL1, BMPR1B, E16, STEAP1, 0772P, MPF, NaPi2b, Sema 5b, PSCA hlg, ETBR, MSG783, STEAP2, TrpM4, CRIPTO, CD21, CD79b, FcRH2, B7-H4, HER2, NCA, MDP, IL20Rct, brevican, EphB2R, ASLG659, PSCA, GEDA, BAFF-R, CD22, CD79a, CXCRS, HLA-DOB, P2X5, CD72, LY64, FcRH1, IRTA2, TENB2, PMEL17, TMEFF1, GDNF-Ra1, Ly6E, TMEM46, Ly6G6D, LGR5, RET, LY6K, GPR19, GPR54, ASPHD1, tyrosinase, TMEM118, GPR172A, MUC16, and CD33.

8. The PROTAC protein according to claim 7, wherein the antibody is a monoclonal antibody or a variant thereof.

9. The PROTAC protein according to claim 8, wherein the monoclonal antibody is selected from the group consisting of trastuzumab, cetuximab, rituximab, brentuximab, gemtuzumab, inotuzumab, sacituzumab, alemtuzumab, and nimotuzumab.

10. A nucleic acid encoding the PROTAC protein according to claim 1.

11. A pharmaceutical composition comprising the PROTAC protein according to any one of claims 1 to 9 or the nucleic acid according to claim 10.

12. The pharmaceutical composition according to claim 11, in which used to treat or prevent cancer or an inflammatory disease.

13. The pharmaceutical composition according to claim 12, in which used to treat or prevent a disease selected from the group consisting of cancer, asthma, autoimmune disease, rheumatoid arthritis, multiple sclerosis, ciliary disease, cleft palate, diabetes, heart disease, hypertension, inflammatory bowel disease, mental retardation, mood disorder, obesity, refractive error, infertility, Engelman syndrome, Canavan disease, chronic digestive disorder, Charcot-Marie-Tooth disease, cystic fibrosis, Duchenne muscular dystrophy, hemochromatosis, hemophilia, Klinefelter syndrome, neurofibromatosis, phenylketonuria, autosomal dominant polycystic neoplasm (PKD1 or PKD2), Prader-Willi syndrome, sickle cell anemia, Tay-Sachs disease, Turner syndrome, HIV-infected disease, and HCV-infected disease.

14. The pharmaceutical composition according to claim 12, wherein the cancer is selected from the group consisting of squamous cell carcinoma, basal cell carcinoma, adenocarcinoma, hepatocellular carcinoma, renal cell carcinoma, bladder cancer, bowel cancer, breast cancer, cervical cancer, uterine cancer, colon cancer, esophageal cancer, head cancer, kidney cancer, liver cancer, lung cancer, ovary cancer, pancreatic cancer, prostate cancer, gastric cancer, leukemia, benign and malignant lymphoma, benign and malignant melanoma, myeloproliferative disease, sarcoma including Ewing sarcoma, angiosarcoma, Kaposi sarcoma, liposarcoma, myoma, neuroepithelial sarcoma, synovial sarcoma, neurosarcoma, astrocytoma, oligodendrogliocytoma, ependymoma, glioblastoma, neuroblastoma, gangliocytoma, ganglioglioma, medulloblastoma, pineocytoma, meningioma, meningeal sarcoma, neurofibroma, and schwannoma, testicular cancer, thyroid cancer, carcinosarcoma, Hodgkin disease, Wilms tumor, and teratocalcinomas.

15. The pharmaceutical composition according to claim 12, wherein the inflammatory disease is selected from the group consisting of arthritis, autoimmune disease, Parkinson's disease, dementia, hepatitis, and viral infection.

16. The pharmaceutical composition according to claim 12, in which administered via oral, parenteral, inhalation spray, topical, rectal, nasal, or implanted reservoir routes.

17. The pharmaceutical composition according to claim 16, in which administered using nanoparticles or liposomes as a carrier upon oral or parenteral administration.
